# EUROPEAN PATENT APPLICATION

(11) **EP 0 695 559 A2**
(43) Date of publication of application: **07.02.1996**
(21) Application number: 95202074.1
(22) Date of filing: 28.07.1995
(51) Int. Cl.: A61N 5/00

(54) **Multifunctional equipment for beauty treatments**

(30) Priority: 05.08.1994 IT MI941711
(71) Applicant: Lazzari, Ambrogio, I-20060 Robbiano di Mediglia (Milano) (IT); Rauli, Donato, I-35035 Mestrino (Padova) (IT)
(72) Inventor: Lazzari, Ambrogio, I-20060 Robbiano di Mediglia (Milano) (IT); Rauli, Donato, I-35035 Mestrino (Padova) (IT)
(74) Representative: Marchi, Massimo

(57) **Abstract**

A multifunctional equipment for beauty treatments of a patient comprises an applicator (1) substantially formed like a sheath, capable of being worn by the patient, that contains at least two pluralities of transducers (6, 7, 8) operating different forms of stimulation; the transducers (6, 7, 8) are operatively connected to a control unit (11) to be operated to execute at least one predetermined treatment programme during which the pluralities of transducers are activated at different times or simultaneously in order to optimize the effectiveness of the treatment.

## Description

The present invention relates to a multifunctional equipment for beauty treatments, particularly electrotherapy, thermotherapy, ultrasonic therapy, cryotherapy, chromotherapy and laser therapy.

Generally, in beauty centers for the treatment of the body of patients, especially for curing cellulitis and other aesthetic defects, several equipments are available, each designed for a given function and suitable for executing a specific treatment, such as electrotherapy, or thermotherapy, or ultrasonic therapy, or cryotherapy, or chromotherapy or laser therapy.

The use of several equipments that operate individually and separately involves a lot of drawbacks and limitations.

The present invention proposes to solve the problems connected with the known equipments by means of a multifunctional equipment capable of executing individual treatments each of which consisting in the application of a stimulation of a given type and multiple treatments consisting in the simultaneous application of stimulations of different type.

According to the invention, a multifunctional equipment for beauty treatments of a patient comprises a control unit and transducers applicable to the body of said patient, said control unit comprising, in turn, means for generating stimulation signals, said transducers being operatively connected to said control unit for executing at least one predetermined treatment programme, characterized in that it comprises an applicator, substantially formed like a sheath, containing at least one plurality of said transducers operating a form of stimulation of a predetermined type and at least another plurality of said transducers operating another form of stimulation of another predetermined type, said applicator being capable of being worn by said patient so that said transducers are automatically positioned in contact with predetermined areas of the body of said patient, said transducers being operatively connected to said control unit to be operated to execute at least one predetermined treatment programme during which said pluralities of transducers are activated at different times or simultaneously in order to optimize the effectiveness of said treatment.

According to a preferred embodiment said applicator comprises a bodice, armlets, an abdominal band and leggings, said bodice, said armlets, said abdominal band and said leggings incorporating said pluralities of transducers and being provided with electrical conductors capable of connecting each of said pluralities of transducers to a respective generator of stimulation signals of a predetermined type.

Preferably, said transducers consist of electrodes for electrotherapy, of electrical resistances for thermotherapy and of emitters of ultrasonic vibrations.

Said transducers can also consist of electrocooling plates for cryotherapy, laser generators, at selected frequencies, for chromotherpay and laser diode probes, operating at the infrared, for laser therapy.

The multi functional equipment according to the invention offers numerous advantages. The main advantage consists in the possibility of executing different treatments simultaneously, with considerable savings in time and with synergy effects, enhancing their effectiveness. Another advantage is represented by the fact that the operations for positioning the transducers and for coupling the electrical connections are simplified and made easy, while the risks of errors are eliminated. Moreover, a single control unit can be connected to several applicators that can be run by a single operator.

Features and advantages of the invention will now be illustrated with reference to an embodiment illustrated as a non-limiting example, in the enclosed figures, wherein:
Fig. 1 shows diagrammatically a multi functional equipment for beauty treatments, made according to the invention;
Fig. 2 shows an applicator, substantially in the form of a sheath, of the equipment of Fig. 1;
Figs. 3 and 4 show control panels of a central unit and of a peripheral unit of a control unit of the equipment of Fig. 1;
Fig. 5 is a diagram that shows treatment programmes that can be executed with the equipment of Fig. 1.

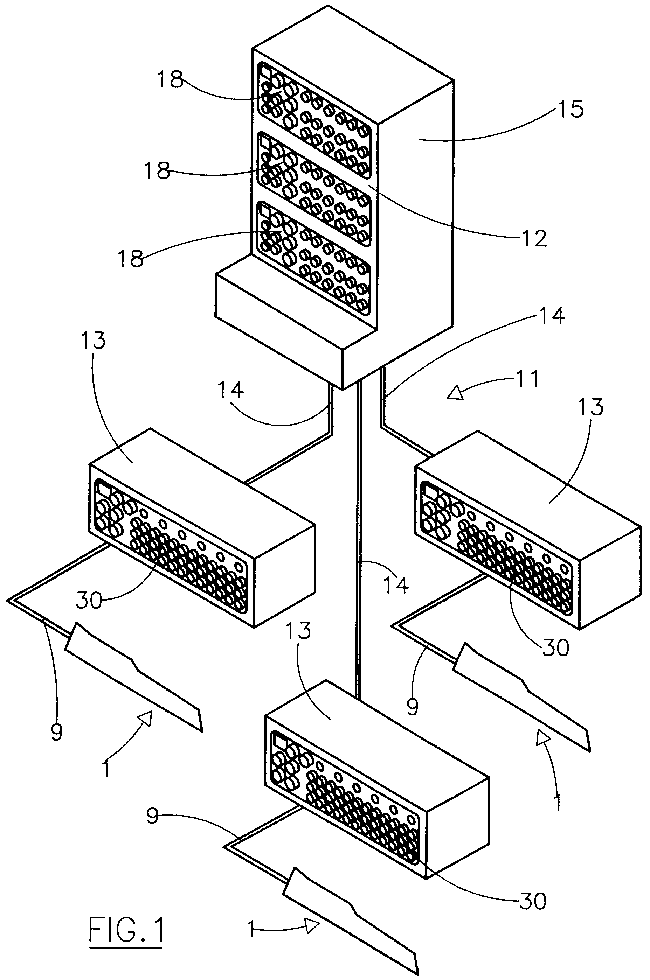

There is shown in Fig. 1 a multifunctional equipment for beauty treatments, made according to the invention, comprising a control unit 11 formed, in the particular case, by a central driving unit 12 and by three peripheral units 13. Each peripheral unit 13 is connected to a respective applicator 1 by means of conductors 9 and to the central unit 12 by means of respective conductors 14.

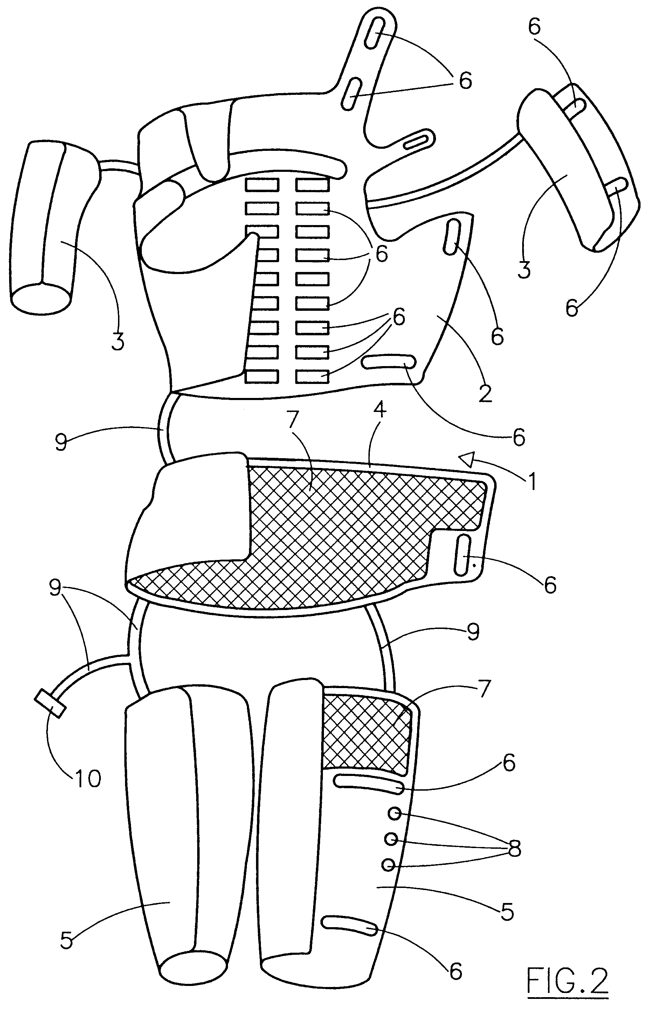

Each applicator 1, as shown in Fig. 2, is substantially formed like a sheath and comprises a bodice 2, armlets 3, an abdominal band 4 and leggings 5, containing pluralities of transducers 6, 7 and 8 operating forms of stimulation of different type. The bodice 2 and the armlets 3 incorporate a plurality of electrodes 6 for electrotherapy, in conductive rubber or other suitable material. The abdominal band 4 incorporates electrodes 6 for electrotherapy and electrical resistances 7 for thermotherapy operating at a low voltage. The resistances 7 are inserted in protective containers of plastic or rubbery material. The leggings 5 incorporate electrodes 6 for electrotherapy, electrical resistances 7 for thermotherapy and emitters 8 of ultrasonic vibrations.

For the sake of clarity, other transducers incorporated in the bodice 2, in the armlets 3, in the band 4 and in the leggings 5 of the applicator 1 are not shown, such as electrocooling plates for cryotherapy, laser generators, at selected frequencies, for chromotherapy and laser diode probes, operating at the infrared, for laser therapy.

The transducers 6, 7 and 8 of the bodice 2, of the armlets 3, of the band 4 and of the leggings 5 are connected to the electrical conductors 9 that, by means of a multiple connector 10, are connected, in turn, to the control unit 11.

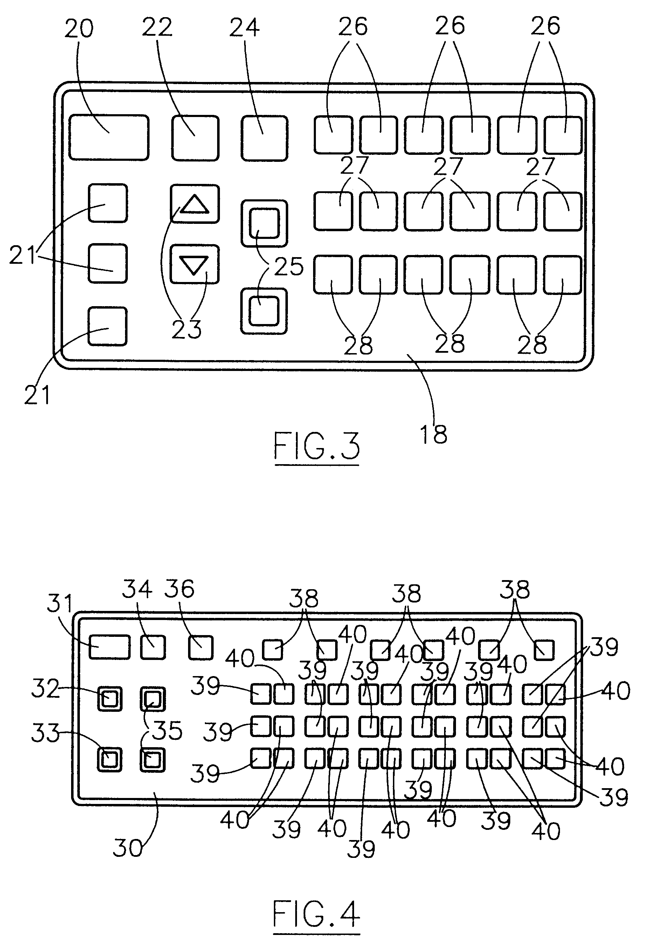

The central unit 12, contained in a box 15, comprises generators of stimulation signals, electrical, ultrasonic, laser, and such like, not shown, as they are known. The generators of stimulation signals, that drive the transducers 6, 7 and 8, are twin, for reasons of safety. The central unit 12 also comprises a programmed microprocessor wherein data are stored related to predetermined treatment programmes that can be executed by means of the transducers 6, 7 and 8. The treatment programmes provide for the pluralities of transducers 6, 7 and 8 to be activated either at different times or simultaneously, according to predetermined sequences. The central unit 12 is provided with three control panels 18, one of which is shown in detail in Fig. 3. A panel 18 comprises a display 20 connected to a timer, that counts the minutes of treatment, pushbuttons 21, that allow the duration of the treatment to be adjusted and a display 22 that indicates the number of treatment programme selected (from 1 to 9) by means of pushbuttons 23. The panel 18 also comprises a display 24, that indicates the state of progress of the programme selected and, in particular, the current step of the programme, consisting of a stimulation of a selected type or of a predetermined combination of stimulations of different type, and leds 25 that indicate the operating conditions of the programme selected. The panel 18 also comprises leds 26, each associated with a given function: stimulation, thermotherapy, ultrasonic therapy, cryotherapy, chromotherapy and laser therapy, pushbuttons 27 for inserting the functions (leds 26 on) and pushbuttons 28 for excluding the same functions (leds 26 off).

Each peripheral unit 13 comprises means for multiplexing, switching and amplifying the stimulation signals that activate the transducers 6, 7 and 8 on the basis of the stimulation signals emitted by the unit 12. The unit 13 is provided with a respective control panel 30 comprising a display 31 that indicates the minutes of treatment, start and stop pushbuttons 32 and 33 and a display 34 that indicates the number of treatment programme selected. The panel 30 also comprises leds 35 that indicate the operating state of the programme selected, and a display 36 that indicates the current step of the treatment programme. The unit 13 also comprises leds 38, each associated with one of the abovementioned functions:
stimulation, thermotherapy, ultrasonic therapy, cryotherapy, chromotherapy and laser therapy, pushbuttons 39 whereby the intensity of the level of the same functions can be selected and leds 40 that display the intensity of the level selected.

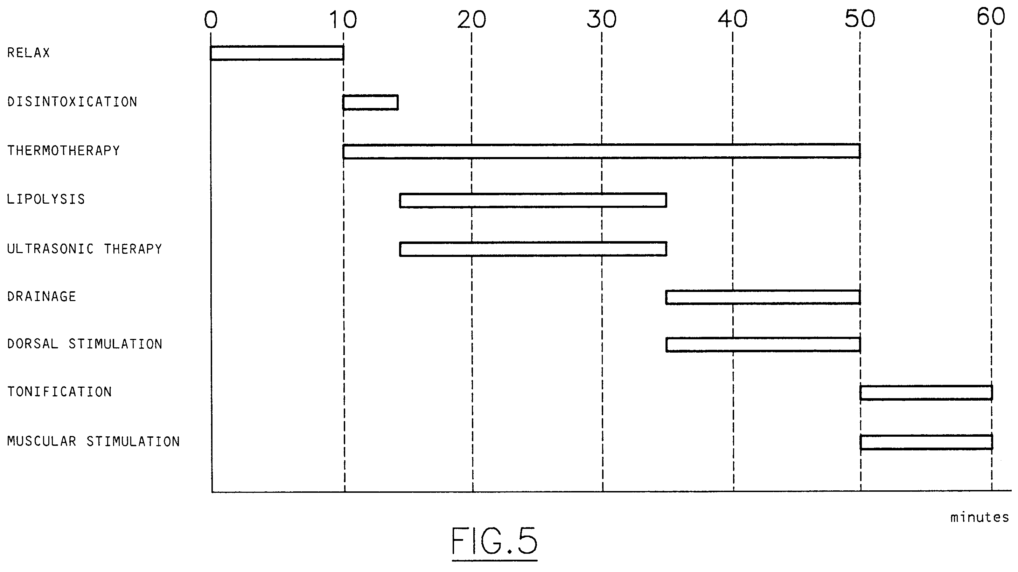

Examples of treatment programmes that can be executed with the equipment of Fig. 1 are shown in the diagram of Fig. 5: relax, disintoxication, thermotherapy, lipolysis, ultrasonic therapy, drainage, dorsal stimulation, tonification, muscular stimulation. In the treatment programmes shown the transducers 6, 7 and 8 are activated either at different times or simultaneously, so as to optimize the effectiveness of the treatment itself. The diagram can be visualised in a special display of the control unit 11 so that the various current steps of the treatment selected can be checked by an operator.

## Claims

1. A multifunctional equipment for beauty treatments of a patient comprising a control unit (11) and transducers (6, 7, 8) applicable to the body of said patient, said control unit (11) comprising, in turn, means for generating stimulation signals, said transducers (6, 7, 8) being operatively connected to said control unit (11) for executing at least one predetermined treatment programme, characterized in that it comprises an applicator (1), substantially formed like a sheath, containing at least one plurality of said transducers (6, 7, 8) operating a form of stimulation of a predetermined type and at least another plurality of said transducers (6, 7, 8) operating another form of stimulation of another predetermined type, said applicator (1) being capable of being worn by said patient so that said transducers (6, 7, 8) are automatically positioned in contact with predetermined areas of the body of said patient, said transducers (6, 7, 8) being operatively connected to said control unit (11) to be operated to execute at least one predetermined treatment programme during which said pluralities of transducers are activated at different times or simultaneously in order to optimize the effectiveness of said treatment.

2. An equipment according to claim 1, characterized in that said applicator (1) comprises a bodice (2), armlets (3), an abdominal band (4) and leggings (5), said bodice (2), said armlets (3), said abdominal band (4) and said leggings (5) incorporating said pluralities of transducers (6, 7, 8) and being provided with electrical conductors (9) capable of connecting each of said pluralities of transducers (6, 7, 8) to a respective generator of stimulation signals of a predetermined type.

3. An equipment according to claim 1, characterized in that said transducers (6, 7, 8) consist of electrodes for electrotherapy (6), of electrical resistances for thermotherapy (7) and of emitters of ultrasonic vibrations (8).

4. An equipment according to claim 1, characterized in that said transducers consist of electrocooling plates for cryotherapy, laser generators at selected frequencies for chromotherpay and laser diode probes, operating at the infrared, for laser therapy.

5. An equipment according to claim 1, characterized in that said control unit (11) comprises a central unit (12) and at least one peripheral unit (13) operatively connected to said central unit (12) and to said pluralities of transducers (6, 7, 8) of said applicator (1), said central unit (12) comprising generators of stimulation signals, electrical, ultrasonic and laser, a programmed microprocessor wherein data are stored related to predetermined treatment programmes and at least one control panel (18), said peripheral unit (13) comprising means for multiplexing, switching and amplifying said stimulation signals and a control panel (30).
